Europäisches Patentamt

European Patent Office

Office européen des brevets

(11) Publication number: **0 319 206**

**A2**

# (12) EUROPEAN PATENT APPLICATION

(21) Application number: **88311183.3**

(22) Date of filing: **25.11.88**

(51) Int. Cl.⁴: **C12N 15/00 , C12N 5/00**

(30) Priority: **30.11.87 US 126436**

(43) Date of publication of application:
**07.06.89 Bulletin 89/23**

(84) Designated Contracting States:
**AT BE CH DE FR GB IT LI LU NL SE**

(71) Applicant: **CODON CORPORATION**
**213 East Grand Avenue**
**South San Francisco California 94025(CA)**

(72) Inventor: **Colby, Wendy W.**
**2018 Lyon Avenue**
**Belmont California 94002(US)**
Inventor: **Morser, Michael J.**
**3964 - 20th Street**
**San Francisco California 94114(US)**
Inventor: **Cashion, Linda M.**
**219 Kelton Avenue**
**San Carlos California 94070(US)**

(74) Representative: **Thomson, Paul Anthony et al**
**Potts, Kerr & Co. 15, Hamilton Square**
**Birkenhead Merseyside L41 6BR(GB)**

(54) **Gene amplification.**

(57) Improved means are provided for obtaining enhanced production of proteins encoded by structural genes of interest in a host based on transfecting the host with an expression vector comprising a wild-type amplifiable gene and the predetermined structural gene. The host is typically not complemented by the amplifiable gene product. If desired, another selection marker may be utilized to select a desired population of cells prior to the amplification.

EP 0 319 206 A2

## GENE AMPLIFICATION

Field of the Invention

This invention relates generally to improved recombinant DNA techniques and the increased expression of mammalian polypeptides in genetically engineered eukaryotic cells. More specifically, the invention relates to improved methods of selecting transfected cells and further, to methods of gene amplification resulting in the expression of predetermined gene products at very high levels.

## BACKGROUND OF THE INVENTION

Expression of cloned genes in bacteria and other prokaryotes has been an important part of genetic engineering research since the mid-1970's. Many gene products, such as insulin, growth hormone, rennin, and alpha thymosin, have been cloned and expressed in E. coli, and the recombinant polypeptide products have been recovered. However, because prokaryotes lack a number of elements contained in eukaryotic cells, such as the eukaryotic signals for transport, modification and glycosylation, significant current research has been directed toward obtaining expression of cloned gene products in eukaryotic, and particularly mammalian cell culture. The ability to produced cloned gene products in mammalian cell culture is particularly important when the cloned gene encodes a eukaryotic polypeptide. In these instances, proper eukaryotic post-translational modification to form a gene product similar to or the same as the natural gene product, is often necessary for normal function of the final recovered protein.

Early experiments of Wigler, M. et al. (Cell (1977) 11:223-232) showed that mammalian cells could be transfected with foreign DNA and that cells having new phenotypes could be isolated. Since transfection procedures with eukaryotic cells are very inefficient, a selection marker is commonly used to isolate those cells which have taken up the foreign DNA. Richard Axel, et al. have used the procedure known as co-transformation, described in the United States Patent No. 4,399,216 and Wigler et al. (Cell (1979) 16,777-785), in which the selectable marker is contained in a plasmid distinct from the foreign DNA.

It is now more common to have the drug selection marker and the non-selectable gene of interest on the same plasmid as separate transcription units, each with their own promoter (Girli, Jouanneau and Yabiv, Virology (1983) 127:385-396, and Southern and Berg, J. Mol. Appl. Genet. (1982) 1:327-341). In the clones isolated from transfection of such plasmids, the expression levels of the non-selectable gene can vary greatly, both in the individual clones and from experiment to experiment.

Typically, a population of cells containing the selectable gene marker is first selected and then screened for the introduction of a non-selectable gene, either by assaying for the non-selectable gene product, or for the presence of the genetic material comprising the coding sequence of the non-selectable gene. This method allows for the stable introduction into cultured mammalian cells of essentially any cloned gene, and the systematic isolation of such cells. Frequently used markers include Herpes viral thymidine kinase (TK), dihydrofolate reductase (dhfr) and rodent CAD. The use of these selection markers is restricted to host cells that do not normally express the selectable gene. Dominant selection markers are more useful for a wide variety of cells, and the following have been generally used: neomycin phosphotransferase from transposon Tn5 (neo), bacterial hygromycin B (hmb), and bacterial XGPRT. The biochemical basis for the selectable markers dhfr, neo, and hmb are described below.

Dihydrofolate reductase (dhfr) is involved in a large number of biochemical pathways, and specifically in thymidine metabolism. MTX can be lethal for the cells if present in the growth medium at high levels, because it binds to dhfr and thereby inactives the enzyme. Thus, methotrexate (MTX) interferes with glycine, hypoxanthine and thymidine metabolism in wild type cultured cells. To overcome the inhibition caused by MTX, the cell may overproduce dhfr protein. One wild-type dhfr gene has been cloned from mouse and its gene product can be used as a selection marker. When taken up by the transfected cell, the additional copies of the dhfr gene lead to increased levels of dhfr protein, which allows growth of the transfected cells in MTX.

The neomycin phosphotransferase gene is derived from transposon Tn5 and confers resistance to aminoglycoside antibiotics, including kanamycin and neomycin, when present in bacteria, or G418 in mammalian cells (Principles of Gene Manipulation, R.W. Old and S.B. Primrose, University of California Press, 2nd ed. (1981). Wild type eukaryotic cells in culture are sensitive to G418, as their growth is prevented if G418 is present in the growth medium. Plasmids containing the neomycin phosphotransferase gene have been used to transfect non-mutant mammalian cell lines, and the transfected cells are then selected by their ability to grow in the presence of G418.

The gene encoding resistance to hygromycin B (hmb) was isolated from E. coli. The gene product is an aminoglycoside phosphotransferase similar to that of neomycin phosphotransferase, but is functional only with hygromycin B. Wild-type bacteria, fungi, and other cultured eukaryotic cells are sensitive to growth in hygromycin B. This sensitivity can be overcome by transfection with plasmids containing a functional hmb gene.

A major issue in this area of research is the need to develop systems which can provide high levels of expression of the gene of interest in a reliable and reproducible way. Several methods to improve gene expression have been used with varying degrees of success.

Gene amplification is a method frequently used to increase expression levels. Gene amplification is often induced by exposure of sensitive cells to stepwise increases in antifolates in the growth medium. Antifolates, such as methotrexate (MTX) and methasquin, are toxic to cells which contain low levels of dihydrofolate reductase or lack it completely. Cultured wild-type cells are sensitive to concentrations of MTX at about 0.1 μM. Resistance to this analog has been found to result from any of several mechanisms: decreased cellular uptake of MTX, overproduction of dhfr, or production of an altered dhfr having lowered affinity for MTX.

The relationship between dhfr gene amplification and MTX resistance in cultures has been well documented (Schimke, Ed. Gene Amplification. Cold Spring Harbor Laboratory, Cold Spring Harbor, NY (1982)). Several groups have shown that while transfected in mutant mammalian cells, cloned dhfr genes can be amplified by MTX selection (Schimke et al., Science (1978) 202:1051-1055, Christman et al, Proc. Natl. Acad. Sci. USA (1982) 79:1815-1819, and Kaufman and Sharp, J. Mol. Biol., (1982) 159:601-621). In addition, DNA sequences linked to these cloned dhfr genes are co-amplified in the same process (Lau and Kan, Proc. Natl. Acad. Sci. USA (1983) 80:5225-5229). It has been found that high MTX resistance by the dose-related phenomenon of gene amplification cannot be obtained by single-step selection protocols. Amplified cells after single-step selections may contain five to ten copies of a gene (Brown, Tisty, and Schimke, Mol. Cell. Biol. (1983) 3:1097-1107). However, by stepwise selection as many as 100 to 1000 dhfr gene copies may be obtained (Schimke, Cell (1984) 37:705-713). It has been shown that not only the dhfr gene is amplified, but also adjacent sequences which can code for other genes. This amplification can yield cells which are resistant to high levels of MTX and have increased levels of production of other associated genes as well (European Patent Applications Nos. 0117059, and 00117060 and Kaufman et al., Mol. Cell. Biol. (1985) 5, 1750-1759, all of which are incorporated herein by reference).

However, gene amplification using antifolates has several key drawbacks, including the instability of resistant clones and the need to grow cultures in the presence of high levels of carcinogens. For example, Kaufman and Sharp (op. cit. (1982)) have shown that when the selective pressure of high levels of MTX is removed, some of the resistant clones are unstable and often lose some of the amplified copies of the dhfr gene, as well as copies of the gene of interest. Although these clones do not usually lose all amplified copies of the genes, this element adds uncertainty to the genetic integrity of the host cell and the yield of the anticipated product. The instability of the clones upon removal of the selective agent can be overcome in some instances by the inclusion of MTX in the production media throughout the fermentation process. But MTX is a potent carcinogen and as such, it is not a desirable component of a large scale fermentation/production system. Further, the presence of methotrexate in the media makes the process of formulation more difficult with respect to health and safety considerations. Therefore, the lower the MTX concentration that is necessary to maintain stability of amplified clones, the greater value those clones have in a commercial production system. Indeed, it would be preferable to develop cell lines that do not require the presence of methotrexate or other selective agents to maintain their high yields.

Mutant derivatives of wild-type dhfr have been isolated and used for selection and amplification of cloned sequences in dhfr- and dhfr+ host cells. These mutations are effective as dominant selection markers by virtue of their reduced affinity for MTX. High levels of MTX can be used to select transformed cells that also express the co-transformed non-selectable gene (EPO Appl. No. 84300300, and Christman et al., op. cit.). However, because the mutant dhfr gene gives resistance to high levels of MTX, amplification may result in only a few copies of the mutant gene rendering cells resistant to high concentrations of MTX (Simonsen and Levinson, Proc. Natl. Acad. Soi. USA (1983) 80:2495-2499). This suggests that amplification of adjacent gene sequences would also be limited to few copies unless extremely high levels of MTX are used. Since the desired end result of amplification is generally high expression of the co-transfected non-selectable gene, and MTX is a carcinogen, this presents a drawback to the use of such a mutant dhfr for amplification of non-selectable co-transfected genes.

The wild type dhfr gene has been conveniently used in some systems to select for amplification of its sequence, as well as for adjacent sequences which can encode other genes. Most dhfr⁻ cells which have been transfected with the dhfr gene are readily subjected to the amplification techniques of growth in antifolates such as methotrexate, as described above (Kaufman et al., Mol. Cell. Biol. (1985) 5:1750-1759).

It has been viewed as a deficiency of the wild-type dhfr as a selectable marker that in tissue culture systems wild-type dhfr confers a relatively low level of MTX resistance upon transfected cells. Although adequate for use in dhfr⁻ cells, this low level of MTX resistance was thought to be too low to constitute a usable dominant selection marker in cells carrying an endogenous wild-type gene (Simonsen and Levinson, op. cit.).

In general, increased production of foreign proteins in a host through gene amplification techniques has been primarily limited to the use of mutant hosts. This is often undesirable for a number of reasons, most notably, that a mutant cellular host suitable for production of the foreign protein is unavailable. The expression of genes into fully active protein commonly requires a specific cell type, which may not be available with the necessary mutation. Moreover, mutating cells will often cause a reduction in growth, protein production or other undesirable charateristics, particularly for high density cell culture.

## SUMMARY OF THE INVENTION

Improved methods are provided for obtaining enhanced production of a structural gene and other desired DNA sequences in a eukaryotic host cell by transfecting the cell with an expression vector comprising a first DNA sequence encoding a wild-type amplifiable gene and a second DNA sequence encoding the structural gene of interest. For expression, each of the first and second sequences are operably linked to one or more regulatory DNA sequences. The host cells, which are typically not complemented by the amplifiable gene product, are grown under conditions that allow selection of cells that have amplified the first DNA sequence. This gene can also be used directly for selecting transfected cells. The amplification also results in increased production of the structural gene. If desired, another selection marker may be utilized to select a population of cells prior to this amplification.

The eukaryotic cell lines useful in the practice of the present invention are wild-type or other cells not auxotrophic for the amplifiable gene on the transfecting plasmid. The amplifiable gene on the expression vector can encode, e.g., dihydrofolate reductase, orthinine decarboxylase, adenosine deaminase, a metallothionein or a CAD gene product. If desired, the plasmid may also contain a selection marker, such as dihydrofolate reductase, neomycin phosphotransferase or, hygromycin B phosphotransferase. The first or second DNA sequences may comprise a single gene or two or more genes. All of these genes may be controlled by the same or different expression systems. In addition, the plasmid vectors may contain bacterial selection markers, regulatory sequences and the like.

Other features and advantages of the present invention will become apparent from the following detailed description, which describes, in conjunction with the accompanying figures and by way of example, the present invention.

## BRIEF DESCRIPTION OF THE FIGURES

Figures 1A-1C are diagramatic gene maps of plasmids pPA 003, pPA 102, pPA 208, pPA 209, pPA 017, pPA 206, pPA 207, pPA 502, pPA 509, pPA 510, and pPA 202.

Figure 2 is a genetic map of plasmid pPA 003. The genetic units of pPA 003 are arranged as indicated on the drawing. The key restriction sites that were used for subsequent constructions are labeled.

### Definitions

dhfr. Refers to the gene encoding dihydrofolate reductase. In this usage dhfr is a selection marker which confers on the cell resistance to methotrexate (Subramani et al., Mol. Cell. Biol. (1981) 1, 854-856). Cells with normal levels of this gene cannot grow in specified amounts of methotrexate, the concentration of which vary among different cell lines.

neo. Refers to the gene obtained from the bacterial transposon Tn5 encoding neomycin phosphotransferase. In this usage neo is a selection marker which confers upon the host cell resistance to a kanomycin, neomycin and G418. Normal eukaryotic cells are unable to grow in media containing 0.5-2.0 mg/ml G418 (Colbere-Garapin et al., J. Mol. Biol. (1981) 150, 1-14).

hmb. Refers to the gene encoding hygromycin phosphotransferase. This gene confers resistance to hygromycin B on the host cell. Cells lacking this gene cannot grow in specified amounts of hygromycin B, the concentration of which will vary among cell lines.

SVe. Refers to the early promoter derived from Simian Virus 40 (SV-40). The promoter used in these studies was a 0.4 Kb Hha I-Hind III fragment which contains the promoter and enhancers regions of the SV40 early genes (Subramani et al.)

LTR. Refers to the long terminal repeat region derived from Harvey murine sarcoma virus (Ha-MSV). The plasmids described in this patent contain three adjacent LTR segments. Each LTR (0.6 Kb) contains a functional promoter and enhancer (Kreigler and Botchan, Mol. Cell. Biol. (1983) 3:325-339).

TK. Refers to the promoter for thymidine kinase from herpes simplex virus.

CHL-1. Refers to a stable transformed cell line used in the disclosed experiments. CHL-1 is a human melanoma cell line capable of unlimited growth in tissue culture systems. These cells are sensitive to defined levels of methotrexate, hygromycin B, and G418. Further, CHL-1 cells are capable of being transfected with exogenous DNA using the calcium phosphate method detailed in this disclosure.

CHL-2. Refers to a stable eukaryotic cell line derived from CHL-1 by transfection with a plasmid encoding t-PA, pPA 003. This plasmid is described in the Detailed Description section.

p    . This is a plasmid designation. "p" written before a set of letters and indicates that what is being referred to is a plasmid, e.g., pPA 509.

t-PA(mU/c/d). Refers to the amount of t-PA produced by a cell in 24 hours. The t-PA is measured by a fibrin plate assay. The assay is described in detailed disclosure of this application. These units are defined by comparison with World Health Organization t-PA international standard.

LTR-tPA. This designation indicates that the plasmid described contains a transcriptional unit comprised of three LTR promoters cloned adjacent to the gene encoding t-PA. This designation also describes a generalized transcriptional unit when written as a promoter-structural gene combination; e.g., TK-hmb, SVe-dhfr. This further indicates that the transcription and translation, i.e., expression, of the t-PA gene is controlled by the LTR promoter and operates in the plasmid in a $5'$ to $3'$ orientation with respect to the t-PA transcription unit.

dhfr-SVe. This designation indicates that the plasmid described contains a transcription unit comprised of the SVe promoter cloned adjacent to the dhfr gene. This designation further should be taken as having the meaning as describing a transcriptional unit when written as a promoter-structural gene combination; e.g., dhfr-SVe. This further indicates that the transcription and translation, i.e., expression of the dhfr gene is controlled by the SVe promoter and operates on the plasmid in a $3'$ to $5'$ orientation with respect to the t-PA transcription unit.


## DETAILED DESCRIPTION OF THE PREFERRED EMBODIMENTS


In accordance with the present invention, novel methods using recombinant expression vectors are provided for increasing the expression levels of a desired protein in a eukaryotic wild-type cell through the amplification of a wild-type amplifiable gene on the expression vector. Such a vector will also include appropriate regulatory sequences for self-replication, and selection in the appropriate host system. The use of these vectors and novel cell lines transfected with the vectors represent a significant improvement over prior expression systems, particularly those that require utilization of mutant genes or auxotrophic cell lines.

In general, the expression vectors of the present invention permit transfection and selection in a eukaryotic cell that carries an endogenous copy of the wild-type amplifiable gene. In some instances, the amplifiable gene can also serve as a selectable marker allowing the transfected cells to be grown in the presence of the selective agent. Most notably, the level of selection agent necessary to cause amplification is typically much lower relative to requirements for a mutant amplifiable gene.

The nature of the host cells will primarily determine the components of the expression vector. Typically, the cells are eukaryotic, preferably human cells, that can grow rapidly in standard media preparations. Substantially any non-microbial cell, whether or not transformed, and which is prevented from growth by a selective agent will find use in the present invention, most notably cells that are not complemented by the amplifiable gene on the expression vector.

The host cell should be capable of rapid growth in cell culture and of glycosylating expressed gene products to ensure that the protein is produced in high quantity yet resembles the naturally occurring material. Cells known to be suitable for dense growth in tissue culture are particularly desirable, and in the art a variety of invertebrate (e.g., insect) or vertebrate cells have been employed, including, cells from toads, mammals, including rodents such as mice, rats, hamsters, rabbits and primates such as monkey or human, whether normal or transformed. These cells will typically be cells of epithelial, endothelial or fibroblastic origin (see, A.T.C.C. Cell Line Register, Rockville, Maryland (1985), which is incorporated herein by reference).

After the host cell has been transfected by standard techniques with the desired expression vector, the media conditions will be manipulated to select amplificants. The precise means of selecting cells that have been amplified will vary depending upon the particular amplifiable gene utilized. Only those cells that have amplified the amplifiable gene will be resistant and survive selective pressure. The cells exhibiting the highest amplification and/or expression of the structural gene can then be cloned for subsequent use. Importantly, as the copy number of the amplification gene increases, so too do the genes linked to it.

For a variety of reasons, the dhfr gene is a preferred amplification gene. In the past, it has been used extensively as a tool to amplify gene sequences of plasmid vectors in mutant cells, primarily those deficient in dhfr. Also, by utilizing plasmids containing both the dhfr gene and a functional structural gene transcription unit, it is possible to generate MTX-resistant clones from normal cells at a very high frequency. Further, the dhfr gene may be used as either a primary or secondary selection marker.

The precise levels of methotrexate needed for selection of clones harboring amplified genes will, of course, vary from one cell line to another, as well as with the culture conditions. For example, in one malanoma cell line clones expressing mouse dhfr were isolated in a limited number of steps (one, two or three) using MTX levels from about 0.05 to 1.0μM. If desired, however, a single MTX selection step at about 0.5μM can yield very high producing clones. In general, because the survival rate at each selection step is low, several steps are preferred to enrich for cell populations that can survive at higher MTX levels.

In addition to dhfr, a number of other amplifiable genes can be utilized. It is preferred, but not necessary, that these genes also serve as direct selection markers in cells that contain an active gene. Also, transfected cell populations can be isolated with a separate marker, preferably a high efficiency gene (e.g., neo or hmb), and then further selected for amplification with the appropriate amplification drug.

The following additional systems are presented by way of example and not limitation. The effective levels of amplifying agent necessary to select for clones containing amplified genes will depend on the agent's toxicity, the culture conditions and cell line in use, but will typically vary from about 0.01μM to about 2.0μM or more.

Ornithine decarboxylase (ODC) is an enzyme that decarboxylates ornithine to produce putrescine, the precurser of the polyamines spermidine and spermine. Cells selected in the presence of high levels of difluormethyl ornithine (DFMO), an inhibitor of ODC, overproduce as much as 1000-fold ODC. (See, McConlogue L. et al, Proc. Natl. Acad. Sci. USA 81:540 (1986), which is incorporated herein by reference.)

Adenosine deaminase (ADA), an enzyme apparently present in all mammalian tissues but not essential for growth, is also suitable for selecting amplificants. ADA catalyzes the irreversible conversion of cytotoxic adenine nucleotides to the respective non-toxic inosine analogs. Thus, when cells are grown in the presence of cytotoxic concentrations of adenosine or adenosine analogs, such as 9-B-D xylofuranosyl adenine (Xyl-A), then ADA is required for detoxification. Thereafter, 2'-deoxycofomycin, a tight-binding transistent state analog inhibitor of ADA, can be added to the growth medium to promote amplification. Importantly, this amplification system can be used with essentially any cell type. (See, Yeung C.-Y. et al, J. Biol. Chem. 258:10299 (1985) and Kaufman, R. et al, Proc. Natl. Acad. Sci. USA, 83:3136 (1986), both of which are incorporated herein by reference.)

A number of other non-selectable genes have been found to be amplified in response to a specific drug incorporated into normal cell culture can be utilized. Typically, expression vectors containing such genes will also contain a separate efficient selection marker, as well as the structural gene to be amplified. A major disadvantage of some of the following systems is the size of the target enzymes, which are often large multifunctional proteins making clones for the entire genes difficult to isolate. However, in some cases, it is possible to substitute in E. coli or other bacterial or yeast enzymes for the functional portion of the mammalian gene. (See, Schimke, R., Cell 37:705 (1984), which is incorporated herein by reference).

CAD is a multifunctional protein which catalyzes the first three reactions of de novo UMP biosynthesis. N-(phosphonacetyl)-L-aspartate (PALA) is a transition state analog inhibitor of the aspartate transcarbamylase (ATCase) activity of CAD. Mutant cells that are resistant to PALA overproduce CAD protein and its mRNA. The mRNA for this protein is greater than about 7.5 kb and only small segments have been presently cloned. A cosmid carrying a 40 kb insert has been used to transfer CAD sequences to wild-type recipients by protoplast fusion. By selecting clones in high levels of PLA, multiple active copies of CAD were found. The ATCase gene from E. coli can be expressed in mammalian cells deficient in ATCase, and selection in PALA results in about a 4-fold amplification of the bacterial sequences. (See, Wahl, G. et al., J. Biol. Chem. 264:8679 (1979), de St. Vincent, B. et al., Cell 27:267 (1981), Padgett, R. et al, J. Mol. Cell. Biol. 2(3):293 (1982), Ruiz, J. et al, J. Mol. Cell. Biol. 6(9):3050 (1986), and Meinkoth, J. et al, J. Somatic. Cell. Genet. 12(4):339 (1986), all of which are incorporated herein by reference.)

UMP-Synthase is a bifunctional enzyme carrying out two activities: orotate phosphoribosyltransferase and orotidine-5'-phosphate dicarboxylates (ODCase). These activities catalyze the conversion of orotic acid to orotadine 5'-monophosphate and orotodine 5'-monophosphate to UMP, the last steps in what result in de novo biosynthesis of UMP. Pyrazofurin monophosphate is a potent inhibitor of the dicarboxylate activity of UMP-Synthase. Cells resistant to pyrasofurin can be selected containing UMP-Synthase levels increased almost 50-fold. Moreover, the amount of UMP-Synthase DNA is increased over normal cells. Similar to the ATCase gene, the ODCase gene from E. coli has been cloned and may be suitable for substitution for the active portion of a mammalian gene. (See, Kanalis, J. et al, J. Biol. Chem. 259:1848 (1984), Kanalis, J. et al, J. Somatic. Cell. Genet. 11(4):359 (1985), Floyd, E. et al, J. Biol. Chem. 260:9443 (1985), and Ohmsted, C. et al, J. Biol. Chem. 261:4276 (1986), all of which are incorporated herein by reference.)

Another suitable gene is 3-hydroxyl-3-methyl-glutaryl (HMG) coenzyme A reductase, which synthesizes mevalonate, essential in the production of cholesterol and of isoprenodes. In a medium lacking lipoproteins, a CHO cell has been developed by adaptation to growth in increasing concentrations of compactin, a noncompetitive inhibitor of reductase. In these cells, the reductase gene has been amplified 15-fold, while the mRNA and the protein are increased 100-fold. The reductase gene product is a transmembrane protein of about 97 kd, and although useful primarily in reductase-deficient cells, is suitable for wild-type cells as well. (See, Chen, D. et al, Nature 308:613 (1984), Reynolds, G. et al, Cell 38:275 (1984), Jil, G. et al, Cell 41:249 (1985), and Chin, D. et al, J. Mol. Cell. Biol. 5:634 (1985), all of which are incorporated herein by reference.)

Asparagine synthetase (AS) is the sole enzyme responsible for the synthesis of asparagine. It is regulated by the concentration of asparagine, as well as by the extent of tRNA aminoacylation. Two analogs are known to inhibit AS in vitro: B-aspartylhydroxamate and albizziin. DNA from mutant CHO cell lines, selected in increasing concentration to drugs, was transfected into mutant cells, and clones were found with AS levels increased 30-fold. The AS subunit has a molecular weight of about 57,000 and is suitable for selection of amplificants wild-type cells. (See, Andrulis, I. et al, Proc. Natl. Acad. Sci. USA 78:5724 (1981), Andrulis, I. et al, J. Mol. Cell Biol. 3:391 (1983), Ray, P. et al, Gene 30:1 (1984), and Andrulis, I. et al, J. Biol. Chem. 260:7523 (1985), all of which are incorporated herein by reference.)

The enzyme adenosinetriphosphatase (ATPase) is known to be overproduced in HeLa cells grown in increasing concentrations of ouabain. ATPase is composed of two subunits, and certain functions reside in the alpha subunit, a 100,000 M.W. polypeptide. Oubain, a cardiac glycocide, binds specifically to the alpha subunit present on the plasma and membrane surface. Cell lines resistant to oubain overproduce ATPase in contained minute chromosomes, a marker for gene amplification. It is believed that the mRNA is amplified about 100-fold. (See, Ash, J. et al, J. Cell. Biol. 99:971 (1984), Emanuel, J. et al, J. Mol. Cell. Biol. 6:2476 (1986), and Pauw, P. et al, J. Mol. Cell. Biol. 6:1164 (1986), all of which are incorporated herein by reference.)

Glutamine synthetase (GS) catalyzes the formation of glutamine from glutamic acid and ammonia. In higher eukaryotes, the enzyme is an octamer of apparently identical subunits. It is known that for certain mammalian cell lines, the specific activity of GS is inversely proportional to the level of glutamine present in the medium. When a population of mouse cells was selected in glutamine-free medium in the presence of the GS inhibitor, methionine sulfoxime, the population overproduced GS and contained high levels of the related mRNA. (See, Young, A. et al, J. Bio. Chem. 258:11260 (1983) and Patejunas, G. et al, J. Mol. Cell. Biol. 7:1070 (1987), both of which are incorporated herein by reference.)

Finally, it is also possible to isolate cells with "amplificator" type or multiple amplification markers. For example, utilizing simultaneous selection with dfhr and CAD genes, clones from BHK cells were selected, subclones of which were again selected for three other amplifiable genes, and the selection rate was about 25 times greater than the original cell. Thus, the use of multiple transfections to introduce multiple amplification markers such as disclosed above, will greatly increase the plasmid copy number and provide even greater expression levels of the desired structural genes. (See, Giulotto, et al, Cell 48:837 (1987), which is incorporated herein by reference.)

Whatever amplifiable gene is chosen, it will be operably linked to one or more regulatory sequences, e.g., the gene will be placed in the expression vector adjacent to a promoter so that expression can occur. Typically, the structural gene will have its own host cell compatible promotor. These promoter/gene cassettes will be adjacent to each other, preferably such that transcription occurs in a divergent manner.

7

The promotors will typically be any of the well known viral promotors derived from Retroviruses, Adenovirus or Simian Virus 40 (SV40). (See, generally, Winnacker, E.-L., Introduction to Gene Technology, VCH Publishers, N.Y. (1987), which is incorporated herein by reference.) The early and late promoters of SV40 are commonly used in CHO cells. In other cells, the Harvey's Sarcoma virus (HaMSC) long terminal repeat (LTR) or any of the well-known divergent promoters are preferred, particularly the former when arranged in a tandem array containing three copies. Generally, the promoter for the amplifiable gene will be weaker than the promoter for the structural gene, in order that greater amplification may be achieved when applying selective pressure yet achieving even greater expression of the structural genes of interest.

As necessary for expression, various prokaryotic and eukaryotic regulatory signals, such as operators, ribosomal binding sites, termination sites, polyadenylation sites and the like will also be operably linked to function in accordance with well known procedures in the art. These regulatory signals are typically derived from viruses, or other DNA sources. The regulatory sequences are usually provided in cassette form in an expression vector, so that the structural gene of interest and the amplifiable gene may be inserted by simple insertion into appropriate enzyme restriction sites.

The expression vector may also contain other regulatory sequences such as any of a variety of prokaryotic replication sequences to allow for amplification in prokaryotic host during preparation of the expression vectors. Thus, for the bacterial cloning, there will be a bacterial selection marker, such as ampicillin, tetracycline, or hmb, that can be used to test for successful transformation and ultimately ensure sufficient quantities of the expression vector for subsequent eukaryotic work.

These vectors also contain, in addition to eukaryotic regulatory sequences, a selection marker so that clones which contain the plasmid can be isolated. Two bacterial genes commonly used are neomycin phosphotransferase from transposon Tn5 and the bacterial gene encoding resistance to hygromycin B. These high efficiency genes generate clones at a very high frequency (greater than about $10^{-3}$ clones/transfected cell). Using these high efficiency markers, individual clones can be isolated from the transfected cell population, but each clone will express different amounts of the foreign gene product. Those skilled in the art will appreciate that the expression vectors can be constructed in a wide variety of means and formats to contain suitable DNA segments. The precise choice and orientation of segments will depend, of course, on the cell host chosen for the ultimate expression of the desired structural gene.

The structural gene may be essentially any DNA sequence (e.g., cDNA or genomic clone with introns) that upon expression codes for a desired polypeptide, including heterologous proteins, exogenous proteins or proteins naturally produced by the host cell. Typically, it will be a eukaryotic structural gene, the protein product of which has a commercial utility, particularly in medical applications. Thus, the polypeptides (typically from about 5,000 to 300,000 MW) can be any variety of proteins, such as enzymes, immunoglobulins, hormones, vaccines, receptors and the like. Examples of proteins falling within the above categories include tissue plasminogen activator, Factor VIII:C, interferons, insulin, growth hormone, growth factors, erythropoietin, interleukins 1, 2, and 3, and others, as new genes are cloned and expressed. Importantly, the entire structural gene for the naturally occurring protein need not be expressed, as fragments or subunits may be produced as desired. If necessary, the proteins will contain leader sequences, transmembrane sequences or the like, depending upon the particular ultimate utility. The precise structural gene incorporated into the expression vector is not crucial to the practice of the present invention.

The following examples are offered by way of illustration and not by way of limitation.

## EXPERIMENTAL

A primary object of the present invention is to provide conditions and plasmids that lead to high levels of production of a predetermined polypeptide.

In the following examples four parameters have been manipulated in order to achieve this goal: the selectable gene marker, the selection conditions, amplification conditions and host cells. The end result from the following experimental work is a production system that will economically produce the desired protein on a large scale. It will be readily apparent to those skilled in the art, that a wide variety of genes of interest may be substituted in place of the structural gene actually utilized (i.e., t-PA).

Design of the Expression Plasmids

The t-PA expression plasmids were constructed with dhfrs genes as selection markers alone or together with neo or hmb genes. These plasmids were used to determine if the dhfr genes could be used as amplification tools and selection markers.

It is a customary practice in this area of research to test various promoters to develop the highest levels of expression for the cloned gene of interest. Some of the more useful promoters include: LTR, the long terminal repeat from Harvey sarcoma virus: TK, the thymidine kinase promoter from Herpes simplex virus; SVe, the SV40 early promoter.

Host Cells

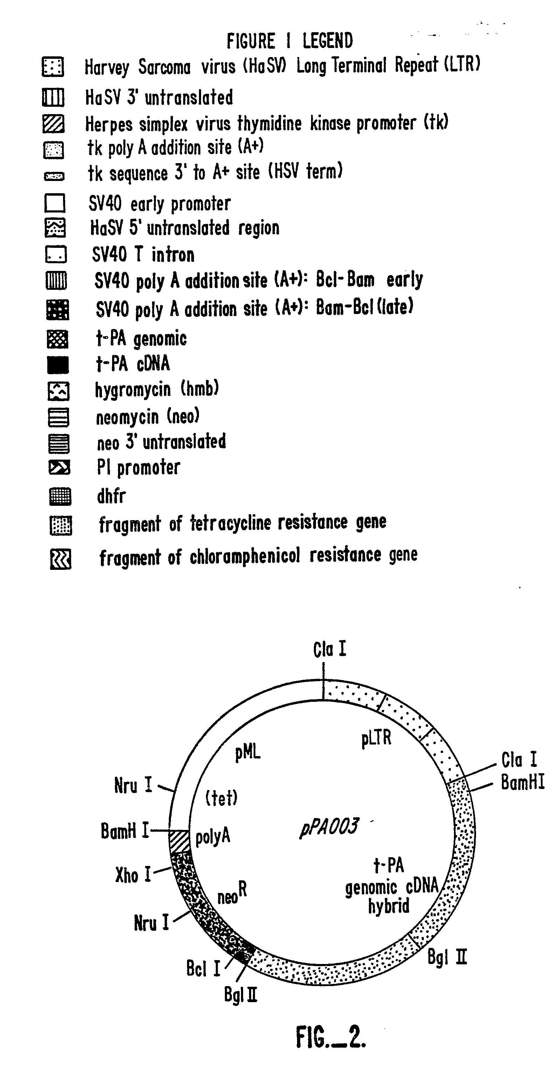

Two different cell lines are utilized. CHL-1 (A.T.C.C. Accession No. CRL 9446) is the cell line used for most of these experiments. A second cell line, CHL-2 (A.T.C.C. Accession No. CRL 9451) is derived from CHL-1 by a previous transfection with a t-PA encoding plasmid, pPA 003 (Fig. 2) which contains the triple LTR promoter, controlling the transcription of both the t-PA gene and the gene encoding neomycin resistance. This stably transfected cell line produces t-PA at a rate of 0.20-0.35 mU/cell/day; about 2-3 times more than the parental CHL-1. CHL-2 was used as a host cell line for some subsequent transfection experiments.

Selection of Clones

Three different selection markers were used to select clones: neo, hmb, or dhfr. T-PA expression plasmids can contain either one or two of these markers.

In the case of plasmids containing only one selection marker, clones were selected and then assayed for expression of t-PA. To compare the expression levels of transfectants generated with these plasmids, a population of clones selected from a single transfection experiment was analyzed to determine average t-PA expression. Individual clones were then isolated from the population to determine the distribution of expression levels of clones within the population. When plasmids containing two selection markers (e.g., hmb and dhfr) were used, a population of clones was isolated first using resistance to hmb and then individual clones were identified by their ability to grow in MTX.

Amplification

Cultures were initially plated into relatively low levels of MTX to select for transfectants that expressed the wild type dhfr gene. Subsequently these cultures were plated into increasing levels of MTX. Clones which had amplified the dhfr gene would confer resistance to the higher levels of MTX. In the process of gene amplification, other plasmid sequences will be co-amplified with the dhfr gene and lead to increased gene expression.

Plasmid Construction

The plasmid pPA 003 is depicted in Fig. 2. It was used in early transfection experiments and then modified as described below to yield new plasmids. The t-PA sequences were inserted into the base plasmid pNeo-5, which was constructed as follows (see, Lusky and Botchan, Cell (1984) 36:391-401 and commonly owned U.S.S.N. 074,083, filed July 16, 1987, which are incorporated herein by reference). The triple eukaryotic promoter from HaMSV was inserted in bacterial plasmid pML. This promoter in the virus is duplicated at both ends of the DNA copy of viral RNA and is contained in the long terminal repeat ("LTR") regions. The TN5 element, containing the neo gene, was obtained from a SV40 hybrid plasmid (Southern and Berg, op. cit.). A signal sequence, which directs polyadenylation of the mRNA of the SV40 T antigen from SV40 DNA, was inserted in the above described plasmid forming plasmid pneo-5.

Plasmid pNeo-5 was used as the basis for a series of expression vectors for t-PA. The DNA sequences coding for t-PA were assembled from two sources: the 5′-end of the gene was from a human DNA gene library and the 3′-end was from a complementary DNA (cDNA) library reverse-transcribed from CHL-1(t-PA)mRNA.

Initially, a cDNA library was constructed from cDNA complementary to CHL-1 mRNA. The CHL-1 cDNA was digested with Bgl II and inserted in Charon 4A, a lambda phage cloning vector (Maniatis, et al., Molecular Cloning, Cold Spring Harbor, 1982, which is incorporated herein by reference). A clone containing the Bgl II fragment which extends from bp 187 to bp 2161 of the coding sequence of t-PA mRNA was identified by hybridization to oligonucleotide probes.

The Bgl II fragment from bp 187 to 2161 was removed from the Charon A4 clone and subcloned into pUC19. Sequences extending only from the Bgl II site at bp 187 to the Xho II site at 1805 were further subcloned by Xho II digestion of this pUC19 derivative and were inserted into the BamH I site of pUC9. Xho II digestion of the pUC9 derivative allowed retrieval of the cDNA fragment. The Xho II fragment was subcloned into the cloning vector p31 at its BamH I and Bgl II sites to produce pPA 106. The Xho II fragment was also subcloned into p31 at its Bgl II site only to produce pPA 104.

In order to express and secrete the mature and active form of t-PA in transfected cells, it was necessary to provide sequences coding for the secretory process. This Bgl II cDNA fragment contains sequences which code for the mature form of the t-PA protein but does not contain sequences for the pre- and pro-peptides of t-PA, that are required for secretion of.the active protein.

In the preferred embodiment, the sequences that encode the pre- and pro- peptides were obtained by isolating a genomic fragment from a human genomic lambda library (Figure 3a). This library was screened using the 414 bp Pst I fragment from the t-PA cDNA as the target sequence on AVX (Maniatis et al.). A 4kb Bgl II genomic fragment was identified that contained the 5' end of the t-PA structural gene including a portion of the 5' untranslated sequences, and 105 nucleotides encoding the pre- and pro-peptides, separated by an intron.

This 4kb Bgl II fragment was subcloned into pPA 106 at its unique Bgl II site to generate the subclone pPA 103. The cDNA/genomic junction was removed by digestion at the Nar I site in the cDNA and a Nar I site in the genomic portion. This Nar I fragment was then moved into the other cDNA subclone, pPA 104 at its equivalent Nar I site (Figure 3b). The new subclone, pPA 115, now contained genomic sequences fused to the cDNA coding region. The hybrid t-PA gene was removed by digesting with Bcl I and partial digestion with Bgl II. The Bcl I-Bgl II cassette was then inserted into the unique Bgl II site of pneo5 thus generating pPA 003. Because the t-PA coding region contains both genomic (including exons and introns) and cDNA sequences, this construction is referred to as a hybrid t-PA gene.

This hybrid t-PA gene then contained the 5' end of the gene including the 5' untranslated sequences of t-PA, the pre- and pro- coding regins, the intron between these regions, and the coding region for mature t-PA.

The hybrid t-PA gene was inserted into the unique Bgl II site in pneo5 to give the expression plasmid pPA 003 (See Fig. 2). In this configuration, the t-PA is expressed along with neo in a dicistronic mRNA using the triple HaMSV LTR promoter region.

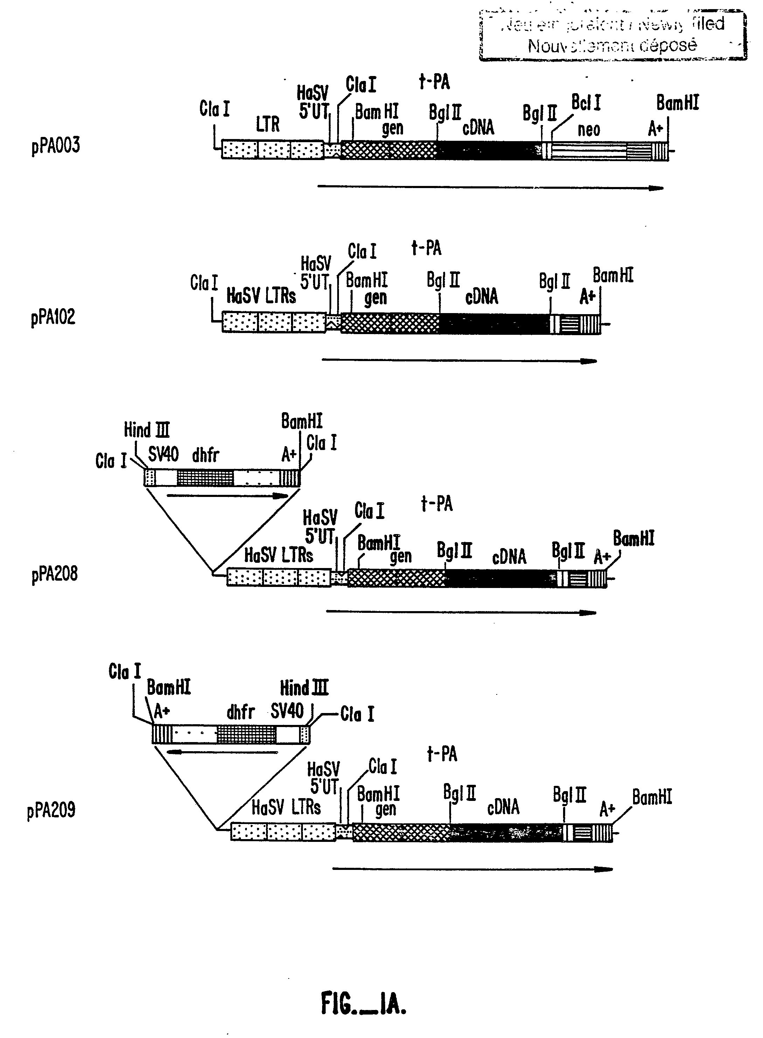

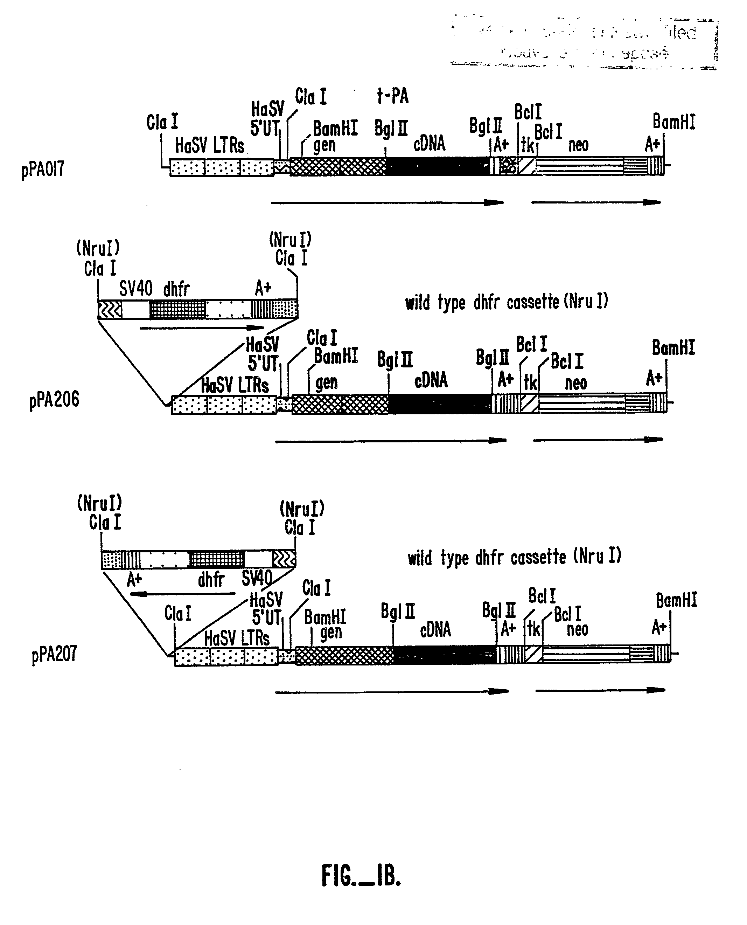

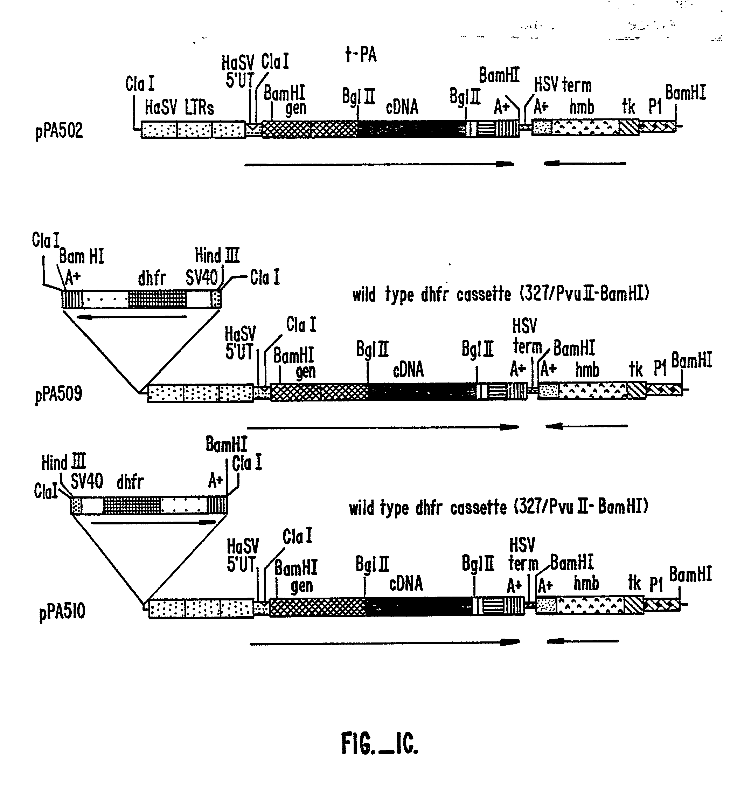

All of the plasmids disclosed were derived from pPA 003, as described and diagramed in Fig. 1.

## PLASMIDS pPA 208 and pPA 209

Plasmids pPA 208 and pPA 209 contain the genomic-CDNA hybrid gene coding for t-PA under control of the triple LTR promoter from Ha-MSV and the wild type dhfr gene under the control of a truncated SVe promoter. The SVe promoter sequence starts at the SV40 Pvu II site and contains a promoter and all but one enhancer region. It is thought to be only slightly less efficient than the SV40 promoter region that extends further to the SV40 Kpn I site (Zenke et al., EMBO (1986) 5:387-397).

The cassette that contains the SVe promoter and dhfr gene was derived as follows. The dhfr gene from PSV2-dhfr (Subramani, Mulligan and Berg, (1982) op. cit.) was removed by digestion with Hind III and BamH I and ligated into the Hind III and BamH I sites of pBR328 (BRL, Bethesda, Md.). SV40 DNA (BRL, Bethesda, MD) was cut at the Hpa II and Hind III sites flanking the SV40 early promoter. This fragment was ligated to the Klenow treated Cla I site and the Hind III site of the pBR 328 vector containing the dhfr gene to produce pSC 614.

Excision of the SV40 promoter and dhfr gene cassette was accomplished by digestion of pSC 614 with pvu II and BamH I. The Pvu II-BamH I fragment was then subcloned into pBR327 which had been cut with EcoR V and BamH I to create pSC 661. PSC 661 was then cut with EcoR I and BamH in order to subclone the SV40-dhfr cassette into a pUC18 derivative, pSC 652, which had also been digested with EcoR I and BamH I. Plasmid pSC 652 was made from pUC18 by putting a Cla I site after the BamH I site using a BamH I-Cla I converting linker. This final subclone was called pSC 662 and has Cla I sites flanking the entire SVe promoter and dhfr cassette.

Plasmid pSC 662 was then digested with Cla I and the SV40 promoter and the dhfr gene were cloned into the Cla I site of pPA 102 which contains the triple HaMSV LTR promoter and t-PA gene. Plasmid pPA 102 was constructed as follows: pPA 003 (described above) was digested at the Bcl I and Xho I sites which flank the neomycin resistance gene. The ends of the DNA were treated with the Klenow fragment of the DNA polymerase, and the blunt ended DNA fragments were religated, thus deleting the neo gene.

Plasmids containing the SVe-dhfr fragment inserted in either orientation were recovered. The dhfr gene transcribed in the same direction as the t-PA gene in pPA 208. In plasmid pPA 209, the dhfr gene is transcribed in the divergent orientation with respect to t-PA.

## PLASMIDS pPA 206 and pPA 207

In plasmid pPA 206 and pPA 207 contain the genomic-cDNA hybrid gene coding for t-PA is under the control of the triple LTR promoter from HaMSV, the selection marker for neomycin resistance under control of the TK promoter, and the wild-type dhfr gene under control of the SVe promoter. To make these plasmids a cassette for the SV40-dhfr sequences were cloned into the parent plasmid pPA 017 which contains the t-PA and neomycin resistance genes.

Plasmid pPA 017 was made by inserting the polyodenylation site from SV40 into pPA 003 at the unique Bcl I site, which is located 3′ to the sequences which encode t-PA mRNA and 5′ to the sequence coding for neomycin resistance. The TK promoter was also inserted into the unique Bcl I site. The Cla I site preceding the triple LTR promoter was cut and an SVe-dhfr cassette with Cla I linkers was cloned into the site in both orientations.

The previously described plasmid pSC614 was used for removal of the cassette. Nru I sites flank the SVe-dhfr sequences in pSC614. Digestion with Nru I produced a blunt ended fragment which contained the SVe-dhfr sequences as well as sequences from the chloramphenicol resistance and tetracycline genes. The SVe promoter in this fragment begins at the Kpn I site rather than the Pvu II site as described in pPA 208 and pPA 209. Cla I linkers were added to the blunt ends on this fragment and the fragment was cloned directly into the Cla I-digested pPA 017 in both orientations.

In pPA 206, the dhfr gene is transcribed in the same direction as the t-PA and neo genes. In pPA 207 the dhfr gene is transcribed in the divergent orientation with respect to t-PA.

## PLASMID pPA 502

Plasmid pPA 502 contains a genomic-cDNA hybrid gene coding for t-PA under control of the triple HaMSV LTR promoter and the gene for hygromycin B resistance under control of the TK promoter. Plasmid pPA 102 described above was used as the source of the LTR-tPA sequences.

The source of the TK-hmb sequences was pHMR272 (Bernard, H.U., Kramma, G., and Rowekamp, W.G.) which contains a bacterial PI promoter followed by the TK promoter, the gene for hygromycin resistance and finally the poly-adenylation region for the TK gene. HMR 272 was digested at its unique Hind III site located at the 3′ end of the TK terminator. The DNA was treated with Klenow fragment and BAM HI linkers were ligated to the linearized DNA. Upon digestion with Bam HI, a DNA fragment 2.7kb in length containing the promoter and hygromycin gene was generated. pPA 102 was partially digested with Bam HI and the Bam HI fragment containing TK-hmb was cloned into the Bam HI site of pPA 102 that follows the t-PA gene and SV40 polyadenylation region. In pPA 502 the orientation of the TK-hmb sequences are such that transcription of hygromycin occurs in the convergent direction to that of t-PA.

## PLASMIDS pPA 509 and pPA 510

Plasmids pPA 509 and pPA 510 contain the wild-type dhfr gene under the control of a truncated SV40 early promoter starting at the SV40 PvuII site, the t-PA genomic-cDNA hybrid gene, and the gene for hygromycin selection. The cloning of this SV40-dhfr cassette is the same as described in that of pPA 208 and pPA 209. This cassette was cloned into the ClaI site of pPA 502 (as described above) directly in front of the triple LTR promoter and t-PA gene. In pPA 509 the dhfr gene is transcribed in the divergent direction from the t-PA gene, while in pPA 510 transcription is in the same direction. The plasmid pPA 509 has been deposited in the ATCC and designated as ATCC No. 67443.

Transfection and Selection of Cells

For each transfection described, 5 pg of plasmid DNA which had been precipitated with calcium chloride by the calcium phosphate method of Wigler et al. (op. cit.) was added to a monolayer of $5 \times 10^5$ host cells in 6 well dishes. Forty-eight hours after transfection, a known number of cells were plated into selective media. In one embodiment, comprising the neo gene, the selection marker confers resistance to G418 at a concentration of 1.0mg/ml. Clones which demonstrated a resistance to 1.0mg/ml G418 were identified and isolated 3 to 4 weeks following transfection. In a second embodiment, resistance to hygromycin is used as a selection marker. CHL-1 cells transfected with the bacterial hmb gene can survive growth in 0.3 mg/ml hygromycin B.

In another embodiment, the selectable phenotype is the expression of dhfr which confers upon the transfected cell the ability to grow in the presence of 100 nM MTX and in the absence of nucleosides. The action of MTX is to inhibit dhfr thus blocking the synthesis of triphosphate nucleosides, precursors of DNA biosynthesis.

The transfection or selection frequency is determined as the number of colonies arising after selection divided by the total number of cells plated.


t-PA Assay

Cells that had taken up plasmid DNA during transfection and were able to grow in the appropriate selection medium formed clones on 100mm petri dishes. These clones were either individually isolated or pooled together as a population in order to assay for t-PA expression.

The assay for t-PA production was carried out in medium containing 0.1% serum and 10 KIU/ml aprotinin. T-PA production in 24 hours was determined for a known number of cells. Aliquots of cell culture supernatant after a 24 hour production period were saved and the number of cells in the culture was counted. For the t-PA analysis, 5 μl samples of culture supernatant were placed in a circular well excised from an agarose matrix containing sheep fibrinogen, human thrombin and human plasminogen. A clearing around the circle of culture supernatant was interpreted as conversion of plasminogen to plasmin which had digested the fibrin, all initiated by the presence of t-PA (Suck, Kabash, and Mannherz Proc. Natl. Acad. Sci. USA (1981) 78:4320-4324). The size of the clearing correlates with the amount of t-PA in the sample. The number of international units was determined by comparison with the cleavage caused by a standard preparation of t-PA that had been calibrated against the t-PA international standard. The values given in the tables are obtained by dividing the t-PA units in the supernatant by the number of cells in the culture.


Amplification Procedure

A population of cells resistant to the first selection conditions (G418 or hygromycin B) were plated into 100nM or 500nM MTX in media lacking nucleosides. Resistant clones were apparent after 4 to 6 weeks. Individual clones resistant to these levels of MTX were isolated and assayed for t-PA expression and a pooled population was plated into higher concentrations of MTX (500nM or 1μM). Clones were again recovered from this selection step and assayed.


EXPERIMENTAL EXAMPLES


Example 1

Plasmid pPA 209 was constructed as shown in Figure 1 and described above. It contains the wild type dhfr gene (as the only selection marker) and the t-PA gene. CHL-1 cells were transfected with pPA 209; pSC614, a dhfr plasmid without t-PA sequences; and salmon sperm DNA as a transfection control. Transfectants were plated into medium lacking nucleosides with 100nM MTX to directly select for clones expressing the plasmid-derived dhfr gene. Untransfected CHL-1 cells cannot survive at that MTX level. The transfection frequencies were determined and individual clones were isolated and assayed for t-PA production. As shown by the transfection frequencies in Table 1, plasmid pPA 209 generates ten-fold more colonies than pSC614, indicating that pPA 209 is an improved construction for use as a dominant selection marker over PSC614, which contains only the SVe promoter and the dhfr gene. The t-PA expression levels of individual clones from pPA 209 are mostly above the background level of the parent cells (0.1 mU/cell/day), showing that this plasmid also directs expression of the non-selected gene. A pooled population of pPA 209 transfectants from 100nM MTX was further selected in 1μM MTX. Clones isolated from 1μM had t-PA production levels which were increased over the expression at 100nM MTX.

These data demonstrate that the wild type dhfr gene provides a useful selection marker when present in a vector, such as pPA 209, for directly isolating transfectants of wild type cells which contain an endogenous dhfr gene. Selection in increased concentrations of MTX leads to isolation of clones with increased expression of the t-PA gene.

Table I

| CHL-1 Transfectants | | | | t-PA Production in mU/cell/day # of clones in each range | | | | |
|---|---|---|---|---|---|---|---|---|
| Selection | Plasmid | Transfection frequency | # of clones assayed | 0-0.2 | 0.3-1.0 | 1.1-2.0 | 2.1-3.0 | 3.1-4.0 |
| 100nM MTX | | | | | | | | |
| | pPA 209 | $2 \times 10^{-4}$ | 25 | 6 | 16 | 2 | 1 | |
| | pSC614 | $2 \times 10^{-5}$ | 7 | 7 | | | | |
| | s. sperm | $2 \times 10^{-5}$ | 8 | 8 | | | | |
| 1uMMTX | | | | | | | | |
| | pPA 209 | | 3 | | | | 1 | 2 |

Example II

The cell line CHL-2 is a derivative of CHL-1 which contains pPA 003, The t-PA production in CHL-2 is increased over CHL-1 from 0.1 mU/cell/day to 0.2-0.3 mU/cell/day. Plasmids pPA 208 (as shown in Figure 1 and discussed above), pPA 209 and pSC614 were transfected into CHL-2 cells. As shown in Table II, both pPA 208 and pPA 209 were improved over pSC614 in generating clones in 100nM MTX. The t-PA expression levels of individual clones of pPA 208 and pPA 209 transfectants from 100nM MTX were well above background levels of the parental cells, indicating these plasmids allow for isolation of clones that express the non-selectable gene. The expression levels of clones generated from pPA 209 in CHL-2 cells are higher than in CHL-1 cells. This process of "supertransfection" of the CHL-2 cells with a secondary plasmid results in t-PA expression levels which are more than additive of the levels which would be expected from each plasmid transfected separately into CHL-1 cells.

This example demonstrates that plasmids with the dhfr gene in opposite orientations can both function as dominant selection markers, and that this selection may also be done in CHL-2 cells which contain an endogenous dhfr gene.

Table II

| CHL-2 Transfectants | | | | t-PA Production in mU/cell/day # of clones in each range | | | | | | |
|---|---|---|---|---|---|---|---|---|---|---|
| Selection | Plasmid | Transfection frequency | # of clones assayed | 0-0.2 | 0.3-1.0 | 1.1-2.0 | 2.1-3.0 | 3.1-4.0 | 4.1-5.0 | 5.1-6.0 |
| 100nM MTX | | | | | | | | | | |
| | pPa 208 | $2.5 \times 10^{-5}$ | 6 | | 2 | | 3 | 1 | | |
| | pPa 209 | $6.3 \times 10^{-5}$ | 5 | 1 | 1 | | | | 2 | 1 |
| | pSC614 | $6.0 \times 10^{-6}$ | 1 | | 1 | | | | | |
| | s. sperm | $1.0 \times 10^{-6}$ | 2 | | 2 | | | | | |

Example III

Plasmids pPA 206 and pPA 207 are t-PA expression plasmids which have two selection markers: neo and dhfr. These plasmids are described above and shown in Figure 1. The dhfr gene is oriented in opposite directions in the pair of plasmids. In the following example, only one selection was used at a time. Following the initial selection in G418, clones were then selected for expression of the dhfr marker.

This experiment was carried out in CHL-1 cells, and transfectants were selected for expression of the neo gene by plating in selective media containing G418. Pooled populations of G418 selected transfectants were then subjected to selection in 100nM MTX. The t-PA expression of mass cultures from both selections is shown in Table III. For each plasmid, the level of t-PA expression is significantly increased after selection in 100nM MTX.

This example demonstrates that the wild type dhfr gene can be used to isolate clones with higher expression levels of the non-selectable t-PA gene in the CHL-1 cell line, which contains an endogenous dhfr gene. The selection for dhfr expression may be subsequent to selection for the neo marker.

Table III

| t-PA Production in mU/cell/day in Mass Cultures of CHL-1 Cells | | |
|---|---|---|
| Selection | pPA206 | pPA207 |
| G418 | 0.19 | 0.30 |
| 100nM MTX | 0.33 | 1.32 |

Example IV

Plasmids pPA 509 and pPA 510 are described above and shown in Figure 1. Each contains two selection markers, hmb and dhfr, along with the t-PA gene. The dhfr gene is oriented in opposite directions in the pair of plasmids. These plasmids were transfected into CHL-2 cells and cells were selected for expression of the hmb gene. Mass cultures and individual clones were assayed for t-PA production which was significantly elevated over the parental cells. The mass cultures were then plated into 100nM or 500nM MTX to select for clones with increased expression of the dhfr gene. Individual clones were isolated from the MTX selectants and mass cultures were also prepared. Analysis of their t-PA production is shown in Tables IV A for the mass culture and IV B for the individual clones. Following selection in 100nM MTX, the expression level of t-PA is increased, but after 500nM MTX selection, expression is increased by an order of magnitude.

This example demonstrates that the wild type dhfr gene can be used to select for clones with much high expression levels of t-PA after a single selection step in MTX. This selection may be subsequent to the selection for the hmb gene and may be done in CHL-2 cells which contain an endogenous dhfr gene.

Table IV A

| t-PA Production in mU/cell/day in Mass Cultures of CHL-2 Cells | | |
|---|---|---|
| Selection | pPA 509 | pPA 510 |
| hygromycin | 1.4 | 1.4 |
| 100nM MTX | 1.4 | 3.0 |
| 500nM MTX | 15.7 | 10.8 |

Table IV B

| CHL-2 Transfectants | | | t-PA Production in mU/cell/day # of clones in each range | | | | | | | |
|---|---|---|---|---|---|---|---|---|---|---|
| Selection/plasmid | Selection frequency | # of clones assayed | 0-1.9 | 2.0-3.9 | 4.0-5.9 | 6.0-7.9 | 8.0-9.9 | 10.0-11.9 | 12.0-13.9 | 14.0-15.9 |
| hygromycin | | | | | | | | | | |
| pPA 509 | $2 \times 10^{-3}$ | 12 | 11 | 1 | | | | | | |
| pPA 510 | $3.6 \times 10^{-3}$ | 12 | 11 | 1 | | | | | | |
| 100nM MTX | | | | | | | | | | |
| pPa 509 | $2 \times 10^{-3}$ | 16 | 5 | 11 | | | | | | |
| pPa 510 | $2 \times 10^{-3}$ | 15 | 1 | 6 | 7 | 1 | | | | |
| 550nM MTX | | | | | | | | | | |
| pPA 509 | $1.2 \times 10^{-4}$ | 22 | | | 3 | 6 | 8 | 2 | 1 | 2 |
| pPA 510 | $5 \times 10^{-5}$ | 29 | 1 | 8 | 15 | 3 | | | 2 | |

EP 0 319 206 A2

Example V

Plasmid pPA 509 was transfected into CHL-1 cells, and clones were selected in hygromycin. A mass culture and individual clones were analyzed for t-PA expression and the mass culture was plated into 100nM MTX. The culture that survived was then subjected to a second round of selection in MTX in either 500nM or 1μM. As shown in Table V, expression of t-PA increases with the concentration of MTX used for the second selection.

This example illustrates that selection for the wild type dhfr gene can be done in a step-wise manner in increasing concentrations of MTX to select for clones with higher expression levels of the t-PA gene. This selection may be done after selection for the hygromycin gene and in cells which contain an endogenous dhfr gene. The amplified genes are stable, thus, eliminating the need for MTX in production.

Table V

| CHL-1 Transfectants | | | t-PA Production in mU/cell/day # of clones in each range | | | |
|---|---|---|---|---|---|---|
| Selection/plasmid | Selection frequency | # of clones assayed | 0-1.9 | 2.0-3.9 | 4.0-5.9 | 6.0-7.9 |
| hygromycin | | | | | | |
| pPA 509 | $6 \times 10^{-4}$ | 24 | 24 | | | |
| 100nM MTX | | | | | | |
| pPA 509 | $2 \times 10^{-3}$ | 4 | 4 | | | |
| 100nM MTX/500 nM MTX | | | | | | |
| pPA 509 | $1 \times 10^{-3}$ | 11 | 7 | 4 | | |
| 100nM/1um MTX | | | | | | |
| pPA 509 | $3 \times 10^{-4}$ | 7 | | 2 | 3 | 2 |

Example VI

A clone isolated after selection in 1μM MTX in example V was recloned by plating at low density in 1μM MTX. Of the single clones isolated, one with high expression levels of t-PA was named W8A5-1. This clone was examined for stability of expression by independent propogation in the presence and absence of the selective agent, MTX. Generation times were calculated at each passage. Cells grew more slowly in 1μM MTX, but in a reduced concentration or in the absence of MTX they grew more rapidly. After 39 generations in a reduced concentration or in the absence of MTX, the level of t-PA production was 74% of that of the culture in 1μM MTX. This illustrates that cells grown in the absence of the selective pressure of MTX do not lose productivity levels of t-PA substantially faster than cells grown in the presence of MTX.

This example demonstrates that the continual presence of MTX in the production phase of a fermentation process is not required to maintain stable expression of a non-selected gene.

Table VI

| Stability of W8A5-1 | | |
|---|---|---|
| MTX conc. | t-PA Production mU/cell/day | Generations |
| 1μM | 11.3 | 29.75 |
| 100nM | 8.7 | 39.0 |
| 0 | 8.4 | 39.25 |

From the foregoing, it will be appreciated that the expression systems of the present invention provide for the increased production of genes in eukaryotic host cells using amplification of wild-type amplifiable genes. Importantly, the host cells need not be mutated or complemented by the amplifiable gene product and, continued selective pressure is not required. This permits the use of a wide variety of cell types for foreign protein production in the absence of potentially harmful selection agents (e.g., MTX). Thus, those cells most suited for high density cell culture can be utilized.

## Claims

1. A method for obtaining increased production of a structural gene in eukaryotic host cells which comprises:
transfecting the cells with an expression vector comprising a first DNA sequence encoding a wild-type amplifiable gene and a second DNA sequence encoding said structural gene, wherein for expression in the host cells each of said first and second sequences are operably linked to one or more regulatory sequences and the host cells are not complemented by such expression; and
growing the cells under conditions suitable for amplifying said first DNA sequence and for selecting transfected cells.

2. A method according to Claim 1, wherein the host cell is a wild-type cell with respect to the amplifiable gene.

3. A method according the Claim 1, wherein the host cell is a human cell line.

4. A method according the Claim 1, wherein the first DNA sequence comprises a single gene.

5. A method according the Claim 1, wherein the amplifiable gene encodes dihydrofolate reductase, a metallothionein, adenosine deaminase, ornithine decarbylase, CAD, UMP-Synthase, asparagine synthetase, adenosinetriphosphatase, glutamine synthetase, and/or HMG coenzyme A reductase.

6. A method according to Claim 1, further comprising a third DNA sequence which comprises a high efficiency selectable gene DNA sequence.

7. A method according to Claim 6, wherein the high efficiency gene encodes neomycin or hygromycin.

8. A eukaryotic cell line containing a plasmid having a first DNA sequence comprising a sequence coding for a wild-type amplifiable gene, a second DNA sequence coding for a structural gene, and one or more regulatory sequences for controlling the expression of said sequences, wherein said cell line is not auxotrophic for the expression product of the amplifiable gene.

9. An improved method for amplifying a DNA sequence encoding a structural gene of interest in wild-type cells comprising introducing into said cells an expression vector having a first DNA sequence comprising a wild-type dhfr gene and a second DNA sequence encoding the structural gene, and growing said cells in the presence of an amplifying concentration of methotrexate.

10. An improved method of selection for and amplifying a DNA sequence encoding a structural gene of interest, an amplifiable gene, and a selectable gene, said method comprising the steps of:
transfecting said DNA sequence in host cells;
growing said cells in the presence of a selective agent; and
growing said selected cells in the presence of an amplifying agent selected from the group consisting essentially of methotrexate, difluormethyl ornithine, 2'-deoxyconfomycin, pyrazofurin monophosphate, albizziin, B-aspartylhydroxamate, ouabain, methionine sulfoxime, and N-(phosphonacetyl)-L-aspartate.

17

FIG._IA.

FIG._IB.

FIG._IC.

## FIGURE I LEGEND

▦ Harvey Sarcoma virus (HaSV) Long Terminal Repeat (LTR)

▥ HaSV 3' untranslated

▨ Herpes simplex virus thymidine kinase promoter (tk)

▦ tk poly A addition site (A+)

▭ tk sequence 3' to A+ site (HSV term)

□ SV40 early promoter

▩ HaSV 5' untranslated region

⬚ SV40 T intron

▥ SV40 poly A addition site (A+): Bcl-Bam early

▦ SV40 poly A addition site (A+): Bam-Bcl (late)

▨ t-PA genomic

■ t-PA cDNA

▨ hygromycin (hmb)

▤ neomycin (neo)

▤ neo 3' untranslated

▨ PI promoter

▦ dhfr

▦ fragment of tetracycline resistance gene

▧ fragment of chloramphenicol resistance gene

FIG._2.